# EUROPEAN PATENT SPECIFICATION

(11) **EP 1 811 027 B1**
(45) Date of publication and mention of the grant of the patent: **27.07.2011**
(21) Application number: 05795112.1
(22) Date of filing: 14.10.2005
(51) Int. Cl.: C12N 15/09, C12P 21/00, A01K 67/033

(54) **POLYNUCLEOTIDE FOR PRODUCTION OF RECOMBINANT PROTEIN BY SILKWORM**
POLYNUKLEOTID ZUR HERSTELLUNG VON REKOMBINANTEM PROTEIN DURCH SEIDENRAUPEN
POLYNUCLEOTIDE POUR LA PRODUCTION D'UNE PROTEINE RECOMBINEE PAR UN VER A SOIE

(30) Priority: 15.10.2004 JP 2004301834
(43) Date of publication of application: 25.07.2007
(73) Proprietor: Hiroshima Industrial Promotion Organization, Hiroshima-shi, Hiroshima 730-0052 (JP); BIOINTEGRENCE INC., Hiroshima-shi, Hiroshima 733-0013 (JP); KOKEN CO., LTD., Toshima-ku, Tokyo 171-0031 (JP)
(72) Inventor: TOMITA, Masahiro, Higashihiroshima-shi, Hiroshima 739-0025 (JP); SHIMIZU, Katsuhiko, Higashihiroshima-shi, Hiroshima 739-0025 (JP); OGAWA, Shingo, Higashihiroshima-shi, Hiroshima 739-0025 (JP); HINO, Rika, Higashihiroshima-shi, Hiroshima 739-0025 (JP); IIZUKA, Masashi, Higashihiroshima-shi, Hiroshima 739-0013 (JP); ADACHI, Takahiro, Higashihiroshima-shi, Hiroshima 739-0044 (JP); YOSHIZATO, Katsutoshi, Higashihiroshima-shi, Hiroshima 739-0144 (JP)
(74) Representative: Gardner, Rebecca Katherine
(86) International application number: PCT/JP2005/019359
(87) International publication number: WO 2006/041225

(56) References cited:
- JP-A- 2002 315 580
- JP-A- 2003 325 188
- JP-A- 2004 016 144
- JP-A- 2004 254 681
- FARRELL P ET AL: "High-level expression of secreted glycoproteins in transformed lepidopteran insect cells using a novel expression vector" BIOTECHNOLOGY AND BIOENGINEERING - COMBINATORIAL CHEMISTRY, WILEY, NEW YORK, NY, US, vol. 60, no. 6, 20 December 1998 (1998-12-20), pages 656-663, XP002143887
- TOMITA MASAHIRO ET AL: "A germline transgenic silkworm that secretes recombinant proteins in the sericin layer of cocoon" TRANSGENIC RESEARCH, vol. 16, no. 4, August 2007 (2007-08), pages 449-465, XP002498415 ISSN: 0962-8819
- OGAWA ET AL: "Generation of a transgenic silkworm that secretes recombinant proteins in the sericin layer of cocoon: Production of recombinant human serum albumin" JOURNAL OF BIOTECHNOLOGY, ELSEVIER SCIENCE PUBLISHERS, AMSTERDAM, NL, vol. 128, no. 3, 24 January 2007 (2007-01-24), pages 531-544, XP005856750 ISSN: 0168-1656
- IIZUKA S. ET AL: 'Chubu Kenshisende Kumikae Tanpakushitsu o Gosei suru Transgenic Kaiko Sakushutsu no Tameno Vector no Kochiku' THE 27TH ANNUAL MEETING OF THE MOLECULAR BIOLOGY TECHNOLOGY SOCIETY OF JAPAN PROGRAM KOEN YOSHISHU November 2004, page 1016, XP002998475
- TOMITA M. ET AL: 'Tanpakushitsu Henseizai o Mochiinakutemo Mayu kara Kumikae Tanpakushitsu o Chushutsu Surukoto ga Kano na Transgenic Kaiko Hatsugenkei no Kaihatsu' THE 27TH ANNUAL MEETING OF THE MOLECULAR BIOLOGY SOCIETY OF JAPAN PROGRAM KOEN YOSHISHU November 2004, page 1016, XP002998476
- MICHAILLE J.J. ET AL: 'Cloning and characterization of the highly polymorphic Ser2 gene of Bombyx mori' GENE vol. 86, 1990, pages 177 - 184, XP002998477
- LU M. ET AL: 'A baculovirus (Bombyx mori nuclear polyhedrosis virus) repeat element functions as a powerful constitutive enhancer in transfected insect cells' J.BIOL.CHEM. vol. 272, 1997, pages 30724 - 30728, XP002184505
- CHEN Y. ET AL: 'A constitutive super-enhancer: homologous region 3 of Bombyx mori nucleopolyhedrovirus' BIOCHEM.BIOPHYS.RES.COMMUN. vol. 318, June 2004, pages 1039 - 1044, XP004508545
- OLSON V.A. ET AL: 'The highly conserved basic domain I of baculovirus IE1 is required for hr enhancer DNA binding and hr-dependent transactivation' J.VIROL. vol. 77, 2003, pages 5668 - 5677, XP002998478
- LU M. ET AL: 'Trans-activation of a cell housekeeping gene promoter by the IE1 gene product of baculoviruses' VIROLOGY vol. 218, 1996, pages 103 - 113, XP002089684

## Description

### Technical Field

The present invention relates to a polynucleotide for producing a recombinant protein in silkworm. More specifically, the invention relates to a polynucleotide for producing a useful recombinant protein in a large amount by silkworm, a vector containing the polynucleotide, and a transgenic silkworm produced using the vector, as well as a process for producing a recombinant protein comprising extracting a useful recombinant protein from cocoons produced by the transgenic silkworm.

### Background Art

Silkworm produces a cocoon immediately before its pupation. Main components of the cocoon are silk proteins and one cocoon contains 0.3 to 0.5 g of silk proteins. The silk proteins are constituted by a protein called fibroin accounting for about 70% and a protein called sericin accounting for the remainder, i.e., about 30%. These silk proteins are synthesized in silk gland. Silk gland is constituted by posterior silk gland, middle silk gland, and anterior silk gland. Fibroin is specifically synthesized and secreted in posterior silk gland and sericin in middle silk gland. Fibroin secreted from posterior silk gland is gradually transferred into middle silk gland by peristaltic movement of silk gland, coated with sericin secreted therein, and further transferred into anterior silk gland to be discharged as a silk filament. Therefore, in the silk filament discharged, fibroin is present in the central part of the filament and sericin is present with surrounding fibroin. Sericin is a protein playing a role of a paste and has a function of adhering discharged silk filaments one another. Although fibroin is extremely insoluble in water, sericin is relatively soluble in water. In the case that raw silk is spun from cocoons, soluble sericin is removed by an operation such as boiling of the cocoons and insoluble fibroin fibers alone are refined as raw silk.

The inventors of the present application have focused ability of synthesizing silk proteins possessed by silkworm and have developed transformed silkworms secreting a large amount of a recombinant protein in a cocoon together with the silk proteins. For the production of a transformed silkworm into which a foreign gene is introduced, there is established a method of injecting to a silkworm egg a minute amount of a plasmid vector into which piggyBac is incorporated, piggyBac being DNA-type transposon derived from a lepidopteran insect Trichoplusia ni (Nat. Biotechnol. 18, 81-84, 2000). They have incorporated a human collagen cDNA linked to downstream of a promoter of a silk protein gene into silkworm using the above gene introducing method to develop a transformed silkworm which produces a recombinant human collagen as part of proteins in a cocoon or silk grand (Non-Patent Document 1) and also have filed patent applications (Patent Documents 1 to 3). Moreover, a patent application on a process for producing a recombinant cytokine has been also filed wherein a transgenic silkworm producing cytokine in silk gland or cocoon filaments is prepared in a similar manner and the cytokine is collected from the silk gland or cocoon filaments (Patent Document 4).

Furthermore, in order to enhance the content of recombinant proteins in a cocoon, the inventors of the present application have focused on a fibroin heavy chain gene having a high transcription activity, specified a polynucleotide as a minimum region which promotes the transcription activity of the gene from its upstream region, proposed a polynucleotide promoting the expression of a foreign gene (Non-Patent Document 2 and 3), and filed a patent application (JP-A-2003-147497).

### List of Background Art

Patent Document 1: JP-A-2001-161214
Patent Document 2: JP-A-2002-315580
Patent Document 3: JP-A-2004-016144
Patent Document 4: JP-A-2003-325188
Non-Patent Document 1: Tomita, M. et al., Nature Biotechnology, 21, 52-56, 2003
Non-Patent Document 2: 25th Annual Meeting of The Molecular Biology Society of Japan, Program/Lecture Abstracts, 2P-1588, Nov. 25, 2002
Non-Patent Document 3: 26th Annual Meeting of The Molecular Biology Society of Japan, Program/Lecture Abstracts, 2PC-174, Nov. 25, 2003

### Disclosure of the Invention

As mentioned above, about 70% of silk proteins constituting a cocoon is fibroin synthesized in posterior silk gland. Therefore, when a recombinant protein gene is expressed in posterior silk gland utilizing a fibroin heavy chain or light chain promoter and an enhancer, a large amount of the protein can be secreted in the cocoon. In this case, since the recombinant protein is embedded in insoluble fibroin fibers, the method is an efficient means for obtaining a mixture of a large amount of the recombinant protein and the cocoon. Moreover, it is also possible to isolate the recombinant protein alone by extracting the recombinant protein from the mixture.

However, since the recombinant protein cannot be extracted from the cocoon unless the insoluble fibroin fibers are dissolved, the extraction and isolation of the recombinant protein from the mixture of the recombinant protein and the cocoon are not always easy. In general, in order to dissolve the fibroin fibers, a mixed solution of a chaotropic salt such as lithium thiocyanate, guanidine thiocyanate, or lithium bromide or calcium chloride and ethanol is used but even when the solution is used, it is difficult to dissolve fibroin fibers completely. Furthermore, when the recombinant protein is extracted using the solution, there is a risk that most of the steric structure of the recombinant protein may be denatured. Therefore, in the case that the recombinant protein has a physiological activity dependent on the steric structure, there is a case that an active recombinant protein is not obtained unless treatment for converting the denatured steric structure into natural one is performed.

Moreover, in order to express a recombinant protein in posterior silk gland and secrete it efficiently in a cocoon, it is considered necessary to synthesize the recombinant protein as a fusion protein with fibroin or a partial sequence of fibroin. This is because endogenous fibroin molecules secreted from posterior silk gland form an extremely dense crystalline structure in the progress of silk filament formation. In the case that the recombinant protein is expressed not as a fusion protein with fibroin but as a single molecule, it is difficult for the recombinant protein to enter between crystals of endogenous fibroin. On the other hand, when it is synthesized as a fusion protein with fibroin, the fibroin part of the fusion protein assists the incorporation into the crystalline structure and the recombinant protein can be embedded in the crystalline structure. As a result, it becomes possible to secrete it into a cocoon. Therefore, the method is an effective means for producing a useful fusion protein comprising, for example, fibroin and a recombinant protein (e.g., silk fibers fused with a recombinant physiologically active protein, etc.) but the necessity of the fusion with fibroin for secretion into a cocoon is not preferable in view of isolating the recombinant protein alone from the cocoon. In order to extract the recombinant protein alone, for example, it becomes necessary to carry out complex and costly steps of inserting a polynucleotide encoding a cleavage sequence of a protease such as factor X or enterokinase between the polynucleotide encoding fibroin and the polynucleotide encoding the recombinant protein beforehand and treating the fusion protein present in the cocoon with factor X and enterokinase.

As above, a method of expressing a recombinant protein gene in posterior silk gland utilizing a fibroin heavy chain and light chain promoter and an enhancer has an advantage that a large amount of the protein can be synthesized but some problems are present in the process of extraction of the recombinant protein.

The invention of the present application is performed in view of the above circumstances and an object of thereof is to provide a novel means capable of solving the problems in the conventional art, secreting a recombinant protein produced in silkworm middle silk gland into a cocoon and extracting the recombinant protein easily without denaturing the steric structure of the protein.

The present application provides the following inventions (1) to (18) as inventions for solving the above problems.
(1) A polynucleotide to be functionally linked to a structural gene for a recombinant protein in an expression cassette for expressing the recombinant protein in silkworm middle silk gland, in which a polynucleotide corresponding to a promoter region of sericin 1 gene or sericin 2 gene is functionally linked with a polynucleotide corresponding to baculovirus homologous regions.
(2) The polynucleotide of the invention (1), wherein the polynucleotide corresponding to a promoter region of sericin 2 gene has the nucleotide sequence of SEQ ID NO: 1 or a part thereof.
(3) An expression cassette for expressing a recombinant protein in silkworm middle silk gland, wherein the polynucleotide of the invention (1) or (2) and a structural gene for the recombinant protein are functionally linked.
(4) A polynucleotide promoting transcription activity possessed by the polynucleotide of the invention (1) or (2), in which a polynucleotide corresponding to a promoter region of sericin 1 gene or sericin 2 gene is functionally linked with a polynucleotide encoding baculovirus IE1 protein.
(5) The polynucleotide of the invention (4), wherein the polynucleotide corresponding to a promoter region of sericin 2 gene had the nucleotide sequence of SEQ ID NO: 1 or a part thereof. ,
(6) A polynucleotide, in which a polynucleotide corresponding to baculovirus homologous regions is further linked with the polynucleotide of the invention (4) or (5).
(7) An expression vector having the expression cassette of the invention (3).
(8) The expression vector of the invention (7), wherein the expression cassette is sandwiched between one pair of inverted repeat sequences of an insect-derived DNA-type transposon.
(9) A vector having the polynucleotide of the invention (4) or (5) or the polynucleotide of the invention (6).
(10) The vector of the invention (9), wherein the polynucleotide is sandwiched between one pair of inverted repeat sequences of an insect-derived DNA-type transposon.
(11) A vector having the expression cassette of the invention (3) and the polynucleotide of the invention (4), (5), or (6).
(12) The vector of the invention (11), wherein the expression cassette and the polynucleotide are sandwiched between one pair of inverted repeat sequences of an insect-derived DNA-type transposon.
(13) A transgenic silkworm, which possesses the expression cassette of the invention (3) in its genome and expresses a recombinant protein in its , middle silk gland.
(14) A transgenic silkworm, which has the polynucleotide of the invention (4), (5), or (6) in its genome and expresses baculovirus IE1 protein in its middle silk gland.
(15) A transgenic silkworm, which has the expression cassette of the invention (3) and the polynucleotide of the invention (4), (5), or (6) in its genome and expresses a recombinant protein in its middle silk gland.
(16) A process for producing a recombinant protein comprising extracting the recombinant protein from cocoons of the transgenic silkworm of the invention (13).
(17) A process for producing a recombinant protein comprising extracting the recombinant protein from cocoons of the transgenic silkworm of the invention (15).
(18) A polynucleotide corresponding to a promoter region of sericin 2 gene, which comprises the nucleotide sequence of SEQ ID NO: 1 or a part thereof.

According to the present inventions, there is provided a polynucleotide capable of expressing a recombinant protein in the state that the protein can be extracted easily from cocoons of the transgenic silkworm without denaturing the steric structure of the protein.

By expressing the recombinant protein in middle silk gland using the promoter and enhancer of sericin, the problems in the case that the recombinant protein is expressed in the posterior silk gland using the promoter region of fibroin, i.e., the problems in the process of extracting the recombinant protein from cocoons can be totally solved. When the recombinant protein gene is expressed in middle silk gland, the synthesized recombinant protein is secreted into a sericin layer soluble in water. Therefore, in order to extract the recombinant protein, it is not necessary to use a solution denaturing the protein, and the recombinant protein can be easily extracted without denaturing it. Even when a recombinant protein having a physiological activity dependent on the steric structure is obtained, it is not necessary to regenerate the steric structure. Furthermore, since endogenous sericin synthesized in middle silk gland does not form a crystalline structure like fibroin, it is not necessary to synthesize the recombinant protein as a fusion protein for the purpose of secreting the protein in a cocoon. Therefore, it is unnecessary to cut off the recombinant protein from a fusion protein by treatment with a protease.

As above, when the recombinant protein is expressed in middle silk gland and secreted into the sericin layer, the problem on the extraction of the recombinant protein from cocoons is solved. However, sericin is a protein constituting 30% of the silk proteins and is present in a smaller amount as compared with fibroin. Therefore, in order to express the recombinant protein gene in middle silk gland and secrete a large amount of the recombinant protein into a cocoon, a polynucleotide as a gene expression-regulating sequence having an extremely high transcription activity in middle silk gland becomes necessary. The invention of the application provides a polynucleotide wherein a polynucleotide constituting a promoter region of sericin 1 or sericin 2 gene is linked to a polynucleotide constituting baculovirus homologous regions as a polynucleotide enhancing the transcription activity possessed by the promoter. Moreover, as a polynucleotide enhancing the transcription activity of the polynucleotide wherein a polynucleotide constituting a promoter region of sericin 1 or sericin 2 gene is linked to a polynucleotide constituting baculovirus homologous regions, the invention further provides a polynucleotide wherein a polynucleotide constituting a promoter region of sericin 1 or sericin 2 gene and a polynucleotide encoding baculovirus IE1 protein are functionally linked.

By using the above polynucleotides, it becomes possible to produce a non-denatured recombinant protein easily in a large amount using silkworm.

In the invention, a "polynucleotide" means a molecule wherein two or more of phosphate esters of nucleotides obtainable by bonding purine or pyrimidine to sugars through a β-N-glycoside bond (ATP, GTP, CTP, UTP; or dATP, dGTP, dCTP, dTTP) are combined. The term that a polynucleotide and another polynucleotide "are functionally linked" means a state that functions possessed by each polynucleotide are not impaired and desired functions can be reserved through linking. Specifically, it means a state that 3'-end of one polynucleotide and 5'-end of another polynucleotide are linked directly or via other linker sequence.

Furthermore, in the invention, a "protein" means a molecule composed of plurality of amino acid residues which are linked to each other through an amide bond (peptide bond) and a "recombinant protein" means a genetically-engineered protein.

In addition, a "structural gene for a recombinant protein" in the invention is a polynucleotide containing a coding region for the recombinant protein (open reading flame: ORF) and is, for example, cDNA of the recombinant protein gene. A "gene promoter region" refers to a region containing a sequence essential for initiating transcription present an upstream region from a transcription starting point of a gene region encoding a protein and to a region generally called a "promoter region" and an "enhancer region".

The other terms and concepts in the invention may be defined in detail in Description of Mode for Carrying Out the Invention and Examples. Moreover, various techniques used for carrying out the invention can be easily and securely carried out based on known literatures and the like except the techniques whose sources are clearly specified. For example, gene engineering and molecular biological techniques are described in Sambrook and Maniatis, in Molecular Cloning-A Laboratory Manual, Cold Spring Harbor Laboratory Press, New York, 1989; Ausubel, F. M. et al., Current Protocols in Molecular Biology, John Wiley & Sons, New York, N.Y, 1995, and the like.

### Brief Description of the Drawings

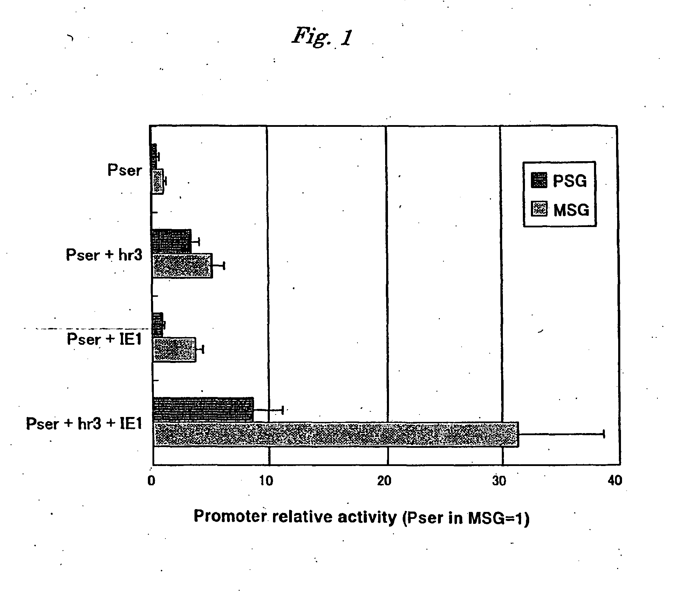

FIG. 1 shows results of investigation of an activity-promoting effect on a promoter of sericin 1 gene by hr3 and IE1 using a transient gene expression system in silk gland using a gene gun. The promoter activity was shown as a relative value when the activity of the sericin 1 gene promoter in middle silk gland is regarded as 1. PSG and MSG represent promoter activity in posterior silk gland and promoter activity in middle silk gland, respectively.

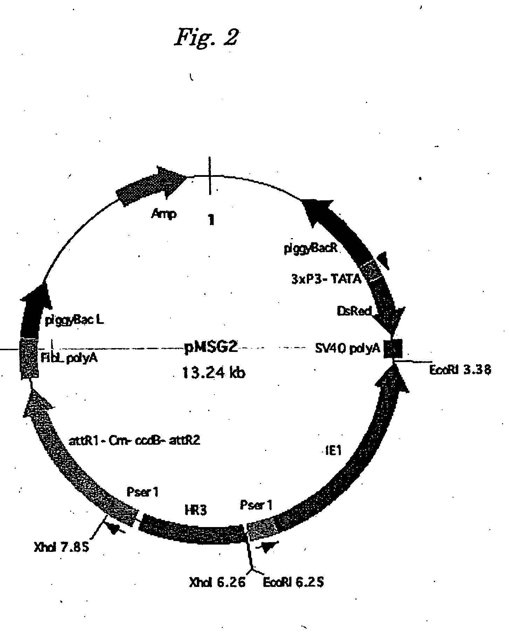

FIG. 2 shows a structure of pMSG2 which is a vector for producing a transgenic silkworm. piggyBacR: sequence at piggyBac 3'-end side, 3xP3-TATA: promoter which induces expression in eyes and neural systems, DsRed: red fluorescent protein gene, SV40 polyA: SV40-derived polyA addition signal, IE1: ORF of BmNPV IE1, Pser1: promoter of silkworm sericin 1 gene, HR3: BmNPV hr3, attR1-Cm-ccdB-attR2: Gateway cassette, FibL polyA: silkworm fibroin L chain polyA addition signal, piggyBacL: sequence at piggyBac 5'-end side.

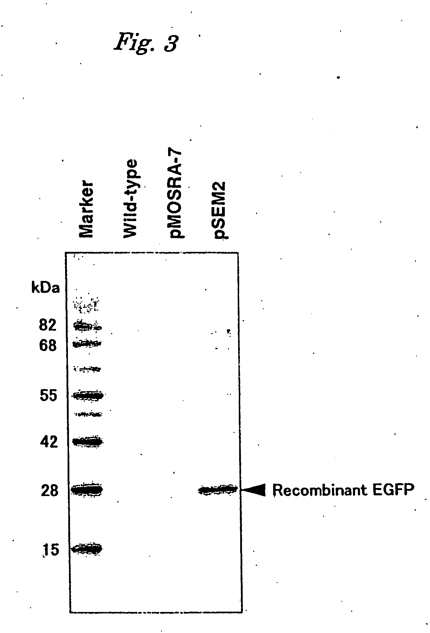

FIG. 3 shows results of Western blotting analysis of cocoon extracts. A cocoon of wild-type silkworm, a cocoon of a transgenic silkworm produced with pMOSRA-7 vector, and a cocoon of a transgenic silkworm produced with pSEM2 vector each was immersed in PBS containing 1% triton-X to extract proteins. After the extracted proteins were subjected to electrophoresis, Western blotting was carried out using an anti-EGFP antibody.

### Best Mode for Carrying Out the Invention

It is known that at least 4 to 6 kinds of molecular species are present as sericin synthesized in middle silk gland of silkworm. These sericin species are synthetic products from two kinds of sericin genes, i.e., sericin 1 gene and sericin 2 gene. Various sericin mRNA's having different sizes are synthesized from each of sericin 1 gene and sericin 2 gene through alternative splicing mechanism and a group of sericin proteins having various molecular weights are translated from these mRNA's (Dev. Biol. 124, 431-440, 1987). In the invention of the present application, a promoter region of sericin 1 gene or sericin 2 gene is used for expressing a recombinant protein in middle silk gland. The promoter region herein refers to a region containing a sequence essential for initiating transcription present an upstream region from a transcription initiating point of sericin 1 gene or sericin 2 gene. Substantially, it refers to a sequence which is a base sequence derived from sericin 1 gene or sericin 2 gene and capable of initiating transcription of a recombinant protein gene linked to downstream of the sequence in middle silk gland cells. So far as it is a sequence satisfying the requirements, the length of the sequence is not restricted. Moreover, it may contain a so-called enhancer sequence which promotes transcription activity of the promoter region. The promoter region of sericin 1 gene can be obtained by a method of designing a primer utilizing a known base sequence (GeneBank/AB007831) or the like and performing genome PCR. The promoter sequence of sericin 2 gene can be obtained by a method of designing a primer utilizing the base sequence (invention (18)) of sericin 2 gene 5' upstream region described in SEQ. I.D: No. 1 cloned by the present inventors using asymmetrical PCR method, a known base sequence (GeneBank/J01036) or the like and subsequently performing genome PCR.

As mentioned above, it is an object of the invention of the present application to achieve the expression of a recombinant protein in middle silk gland and express a large amount of the recombinant protein. According to the inventions precisely described in the following, transcription activity possessed by a promoter of sericin 1 gene or sericin 2 gene in middle silk gland cells are enhanced.

The inventions (1) and (2) are polynucleotides wherein transcription activity possessed by a promoter of sericin 1 gene or sericin 2 gene in middle silk gland cells is enhanced by combining a polynucleotide constituting a promoter region of sericin 1 gene or sericin 2 gene with a polynucleotide constituting baculovirus homologous regions (hereinafter, referred to as "hrs" or sometimes "hr" in the singular case). The "hrs" is a certain kind of repeating sequence scattered in genome of baculovirus (BmNPV, AcNPV, CfNPV, LdNPV, OpNPV, etc.) and acts as a duplication base point of virus DNA as well as has a function as an enhancer of promoting transcription activity of various genes present in baculovirus genome (J. Biol. Chem. 272, 30724-30728, 1997). Moreover, hrs is known to act as an enhancer for transcription of genes other than baculovirus genes. For example, hr3 which is one of hrs of BmNPV acts on a silkworm actin promoter (J. Biol. Chem. 272, 30724-30728, 1997) and hr5 which is one of hrs of AcNPV acts on LTR of Rous sarcoma virus (J. Virol. 61, 2091-2099, 1987), and it is reported that they enhance transcription activity thereof. However, it is not known whether hrs can enhance transcription activity possessed by a promoter of sericin 1 gene or sericin 2 gene in middle silk gland or not. Thus, this point was investigated in the following Example 1. As a result, hrs is found to enhance transcription activity possessed by a promoter of sericin 1 gene or sericin 2 gene in middle silk gland and thus the inventions (1) and (2) have been accomplished. The hr to be used in the invention of the application may be derived from any baculovirus so far as it has an effect of enhancing the activity of a promoter of sericin 1 gene or sericin 2 gene and may be any kind of hrs, while at least 9 kinds are known as hrs. Furthermore, it may be a partial sequence of hrs so far as it has an effect of enhancing the activity of a promoter of sericin 1 gene or sericin 2 gene. These hrs or partial sequences of hrs can be obtained by a method of designing a primer utilizing known base sequences (GeneBank/NC_001962, GeneBank/NC_001623, etc.) and performing PCR using a virus genome as a template.

The invention (3) is an expression cassette wherein the polynucleotide of the invention (1) and a structural gene of the recombinant protein are linked. As the structural gene of the recombinant protein in the cassette, a cDNA encoding any protein or the like may be used. When the recombinant protein is a secretory protein, a cDNA encoding the protein may be used as it is. When it is not a secretory protein, a sequence encoding a signal peptide may be added to the 5'-end side of the cDNA. The sequence encoding a signal peptide may be derived from silk proteins such as sericin or may be derived from other any secretory protein. In the expression cassette of the invention (3), the promoter region of sericin 1 gene or sericin 2 gene is necessarily linked to upstream of the structural gene of the recombinant protein but hrs may be located at upstream of the polynucleotide comprising the promoter region of sericin 1 gene or sericin 2 gene and the structural gene of the recombinant protein or located at downstream thereof. Moreover, it may be linked adjacent to the polynucleotide comprising the promoter region of sericin 1 gene or sericin 2 gene and the structural gene of the recombinant protein or may be linked apart therefrom so far as the distance is within an effective range.

The inventions (4) and (5) of the application are polynucleotides for further enhancing the transcription activity possessed by the polynucleotide of the invention (1). The polynucleotides are composed of a polynucleotide constituting a promoter region of a sericin 1 or sericin 2 gene and a polynucleotide encoding baculovirus IE1 protein linked to downstream thereof. IE1 is one of a group of proteins synthesized immediately after a host cell is infected with baculovirus and is a transregulator activating expression of virus genes such as 39k and p35 and ie1 gene itself encoding IE1 protein (J. Virol. 57, 563-571, 1986, J. Virol. 66, 7429-7437, 1992). It is known that the mechanism of activation of the expression of these virus genes induced by IE1 involves a mechanism of enhancing the transcription activity of the promoter via hrs and a mechanism of directly acting on the promoter without the intervention of hrs (J. Virol. 77, 5668-5677, 2003). Moreover, IE1 is known to activate the expression of genes of the host cell such as actin gene. (Virology 218, 103-113, 1996). The inventors of the present application have considered a possibility that IE1 protein may enhance the transcription activity possessed by the polynucleotides of the invention (1) and (2) in middle silk gland cells and have investigated the possibility in the following Example 1. As a result, it has clarified that IE1 protein enhances the transcription activity possessed by the polynucleotides of the invention (1) and (2) in middle silk gland cells by both of the mechanism of enhancing the transcription activity of the promoter via hrs and the mechanism of directly acting on the promoter without the intervention of hrs. Thus, a polynucleotide wherein a promoter of sericin 1 gene or sericin 2 gene has been linked to upstream of ORF encoding IE1 protein has been then prepared so that IE1 protein has been synthesized in middle silk gland, and thereby the inventions (4) and (5) have been accomplished. Furthermore, baculovirus hrs has been linked to the polypeptides of the inventions (4) and (5) to develop a polynucleotide capable of synthesizing a larger amount of IE1 protein (invention (6)). In the invention (6), hrs may be linked to upstream of the polynucleotide of the invention (4) or (5) or linked to downstream thereof. Moreover, it may be linked adjacent thereto or may be linked apart therefrom so far as the distance is within an effective range. The ORF encoding IE1 protein in the inventions (4) and (5) and the invention (6) can be obtained by a method of designing a primer utilizing a known base sequence (GeneBank/AY048770, GeneBank/M 16820) or the like and performing PCR using a virus genome as a template. The ORF encoding IE1 protein may be derived from any baculovirus so far as synthesized IE1 has an effect of enhancing the transcription activity of the promoter region of sericin gene 1 or 2 and the polynucleotides of the inventions (1) and (2) and may be a partial sequence of IE1 or one wherein a part of the base sequence is modified.

The invention (7) is an expression vector possessing the expression cassette of the invention (3). Moreover, the invention (9) is a vector possessing the polynucleotide of the invention (4), (5) or (6). These vectors can be used without particular limitation so far as they are vectors for insects capable of use for transformation of silkworm. For example, they may be AcNPV vector, a plasmid vector into which insect-derived DNA-type transposon, or the like but the latter is particularly preferred (invention (8) and invention (10)). As the insect-derived DNA-type transposon, piggyBac, mariner (Insect Mol. Biol. 9, 145-155, 2000), Minos (Insect Mol. Biol. 9, 277-281, 2000), and the like are known. Since these transposons show migration activity in silkworm cells, it is possible to transform silkworm with a vector prepared based on the DNA-type transposon. In particular, a plasmid vector based on piggyBac has successfully actually transformed silkworm by injecting a minute amount thereof into silkworm eggs (Nat. Biotechnol. 18, 81-84, 2000).

The invention (11) is a vector possessing the expression cassette of the invention (3), i.e., both of the polynucleotide for expressing the recombinant protein and the polynucleotide of the invention (4), (5) or (6), i.e., the polynucleotide for expressing IE1. Moreover, the invention (12) is a vector prepared based on an insect-derived DNA-type transposon, which is one embodiment of the invention (10). In these vectors, the expression cassette expressing the recombinant protein and the polynucleotide synthesizing IE1 may be possessed completely independently in one vector or may share a promoter or hrs. For example, when they are incorporated into the vector in the order of recombinant protein gene-promoter-hrs-promoter-IE1ORF, hrs can be shared. Moreover, when they are incorporated, using IRES (Internal Ribosome Entry Site) which functions in silkworm silk gland cells, for example, in the order of hrs-promoter-recombinant protein gene-IRES-IElORF, hrs and the promoter can be shared.

A transgenic silkworm (invention (13)) which possesses the expression cassette for expressing the recombinant protein in its genome can be produced using the vector of the invention (7) or (8), and a transgenic silkworm (invention (14)) which possesses the polynucleotide expressing IE1 in its genome can be made using the vector of the invention (9) or (10). Moreover, when the vector of the invention (11) or (12) is used, a transgenic silkworm (invention (15)) which possesses both of the expression cassette for expressing the recombinant protein and the polynucleotide expressing IE1 in its genome can be produced.

With regard to the production of the transgenic silkworm, in the case of utilizing the vector prepared based on piggyBac, for example, the production can be carried out by a method similar to the method of Tamura et al. (Nat. Biotechnol. 18, 81-84, 2000). Namely, one pair of inverted repeat sequences of piggyBac is incorporated into an appropriate plasmid vector and a polynucleotide to be inserted is inserted so as to be present between one pair of the inverted repeat sequences. Then, the plasmid vector is microinjected into silkworm eggs together with a transposase expression vector (helper plasmid) of piggyBac. The helper plasmid is a recombinant plasmid vector wherein only the transposase gene region of piggyBac is substantially incorporated with lacking one or both of the inverted repeat sequences of piggyBac. In the helper plasmid, an endogenous transposase promoter may be utilized as it is or a silkworm actin promoter, drosophila HSP70 promoter, or the like may be utilized as the promoter for expressing the transposase. In order to facilitate screening of next-generation silkworm, it is possible that a marker gene has been simultaneously incorporated into the vector into which the polynucleotide to be inserted has been incorporated. In this case, a promoter sequence such as the silkworm actin promoter or drosophila HSP70 promoter is, for example, incorporated into upstream of the marker gene and the marker gene is expressed by the action. An insulator may be incorporated between the promoter expressing the marker gene and hrs so that the promoter expressing the marker gene is not affected by hrs present in the vector. As the insulator to be used, an insulator of drosophila gypsy transposon or the like may be mentioned, for example.

Larvae (F0 generation) incubated from the silkworm eggs into which the vector have been microinjected are raised. The resulting all silkworms of F0 generation are crossed with wild-type silkworms or the F0 silkworms are crossed with each other and transgenic silkworms are selected from silkworms of next-generation (F1 generation). The selection of the transgenic silkworms is carried out using a PCR method or Southern plotting method, for example. Moreover, in the case that a marker gene is incorporated, it is possible to select the silkworms utilizing the expressed trait. For example, in the case that a fluorescent protein gene such as GFP is utilized as the marker gene, the selection can be carried out by irradiating silkworm eggs and larvae of F1 generation with an excitation light and detecting fluorescence emitted from the fluorescent protein. A transgenic silkworm can be produced by the method mentioned above.

The transgenic silkworms of the inventions (13) and (15) express a recombinant protein in middle silk gland and secrete the recombinant protein in the sericin layer of silk filaments. Since a polynucleotide expressing IE1 is incorporated into the transgenic silkworm of the invention (15) in addition to the expression cassette for expressing the recombinant protein, a larger amount of the recombinant protein is expressed as compared with the transgenic silkworm (invention (13)) into which only the expression cassette for expressing the recombinant protein is incorporated. The transgenic silkworm of the invention (15) can be produced using the vector of the invention (11) or (12) as mentioned above or may be produced by incorporating a polynucleotide expressing IE1 using the vector of the invention (9) or (10) into the transgenic silkworm of the invention (13) into which the expression cassette for expressing the recombinant protein is incorporated or by incorporating the expression cassette for expressing the recombinant protein using the vector of the invention (7) or (8) into the transgenic silkworm of the invention (14) into which the polynucleotide expressing IE1 is incorporated. Moreover, the transgenic silkworm can be also produced by crossing the transgenic silkworm of the invention (13) with the transgenic silkworm of the invention (14) and selecting a silkworm possessing both of the expression cassette for expressing the recombinant protein and the polynucleotide expressing IE1 from next-generation silkworms.

The inventions (16) and (17) are processes for producing a recombinant protein comprising extracting the recombinant protein from cocoons of the transgenic silkworms of the inventions (13) and (15), respectively. The recombinant protein synthesized by the transgenic silkworm of the invention (13) or (15) is secreted to the sericin layer of silk filaments constituting cocoons. As mentioned above, the sericin layer is composed of sericin soluble in water and the recombinant protein localized in the layer can be extracted without using a solution which denatures proteins. The extracting liquid for extracting the recombinant protein from the sericin layer is not particularly limited so far as it is capable of extracting the recombinant protein. For example, it may be a neutral salt solution or may be a solution containing a surfactant and other reagent(s) for efficient extraction, and the like. For extracting the recombinant protein from cocoons using these extracting liquids, a method of immersing fragmented cocoons in the extracting liquid and stirring the whole can be employed. Moreover, before the extraction, the cocoons may be subjected to pulverizing treatment and also mechanical treatment such as ultrasonic treatment at the extraction may be employed in combination.

### Examples

The following will describe the invention further in detail with reference to Examples but the invention is not limited to the following examples.

### Example 1

### Verification of effect on promotion of activity of promoter of sericin 1 gene by hr3 and IE1

The following three kinds of vectors were prepared and an effect of promoting transcription activity of silkworm sericin 1 promoter by hr3, which is one of hrs of BmNPV, and IE1 of BmNPV was investigated utilizing a temporary gene expression system using a gene gun.

### [1] Vector having firefly luciferase gene at downstream of sericin 1 promoter

A DNA fragment corresponding to the region of -304- +20 when a transcription initiating point of silkworm sericin 1 gene was regarded as + 1 was obtained by PCR using genome DNA extracted from adult silkworm abdomen as a template. The primers used were 5'-GCTAGCAGTCGAATTTCGACTACTGCG-3' (SEQ ID NO: 2) and 5'-GCTAGCCCCGATGATAAGACGACTATG-3' (SEQ ID NO: 3) and an NheI site was added to each 5'-end. The resulting PCR product was cleaved with Nhel and then inserted into an NheI site of firefly luciferase reporter vector pGL3-basic (Promega).

### [2] Vector having firefly luciferase gene at downstream of hr3 and sericin 1 promoter

A DNA fragment containing hr3 (base Nos. 64321-66017: GeneBank database registry No. NC_001962) of BmNPV was obtained by PCR using pXINSECT-DEST38 (Invitrogen) as a template. The primers used were 5'-CGGAATCTATGTTACGGACTTC-3' (SEQ ID NO: 4) and 5'-GAGCTCGATATCGAATTCCTGCAGCC-3' (SEQ ID NO: 5: an SacI site was added to 5'-end). The resulting PCR product was cleaved with SacI and then inserted into an SacI site at upstream of sericin promoter of pGL3 having sericin 1 promoter of the above [1]. The direction of the inserted DNA fragment was made so that the 5'-end (base No. 64321) side of hr3 was toward the sericin 1 promoter side.

### [3] Vector having ORF of IE 1 at downstream of hr3 and sericin 1 promoter

An ORF (base Nos. 116981-119482: GeneBank database registry No. NC_001962) of IE1 of BmNPV was obtained by PCR using pXINSECT-DEST38 as a template. The primers used were. 5'-GGATCCCAACCAAACGACTATGACGC-3' (SEQ ID NO: 6: a BamHI site was added to 5'-end) and 5'-CAGGAGTGGGCATACTCTTG-3' (SEQ ID NO: 7). The resulting PCR product was inserted into pCR4Blunt-TOPO vector (Invitrogen). Then, sericin 1 promoter is amplified by PCR from pGL3 vector having sericin 1 promoter of [1]. The primers used were 5'-GGATCCGAGCTCAGTCGAATTTCGACTACTGCG-3' (SEQ ID NO: 8: a BamHI site and an SacI site were added to 5'-end) and 5'-GGATCCGCTAGCCCCGATGATAAGACGACTATG-3' (SEQ ID NO: 9: a BamHI site was added to 5'-end). The PCR product was digested with BamHI and inserted into a BamHI site present at 5'-end side of IE1ORF of the above pCR4Blunt-TOPO vector having IE1ORF. Finally, by digesting the vector of [2] having firefly luciferase gene at downstream of hr3 and sericin 1 promoter with SacI, hr3 was cut out of the vector and was inserted into the SacI site present at 5'-end side of sericin 1 promoter of the above vector having sericin 1 promoter and IE1ORF. The direction of the insertion was made so that the 3'-end (base No. 66017: GeneBank database registry No. NC_001962) side of hr3 was toward the sericin 1 promoter side.

A DNA solution obtained by mixing the above vector of [1] and pCR4Blunt-TOPO vector containing no inserted DNA in the ratio of 1:1 (hereinafter referred to as Pser), a DNA solution obtained by mixing the above vector of [2] and pCR4Blunt-TOPO vector containing no inserted DNA in the ratio of 1:1 (hereinafter referred to as Pser+hr3), a DNA solution obtained by mixing the above vector of [1] and the above vector of [3] in the ratio of 1:1 (hereinafter referred to as Pser+IE1), and a DNA solution obtained by mixing the above vector of [2] and the above vector of [3] in the ratio of 1:1 (hereinafter referred to as Pser+hr3+IE1) were prepared. According to the method described in the following, these four kinds of DNA mixed solutions were introduced into silkworm silk glands and luciferase activity was measured.
1) Preparation of gold particles: A DNA mixed solution was attached in an amount of 188 ng per mg of gold particles.
2) Collection of silk gland: A silk gland was taken out of a larva at 5th stage 1 day and washed with Grace medium.
3) Introduction of DNA into silk gland by means of gene gun: Using a gene gun (Helios Gene Gun; BioRad), the gold particles to which DNA had been attached were injected into the silk gland. The gold particles (3.8 ng DNA) were injected in an amount of 0.02 mg per silk gland.
4) Transplantation of silk gland: After the silk gland was washed with Grace medium, it was transplanted into backside posterior coelom of a larva of the age in day the same as that of the larva, out of which the silk gland was taken. The larva into which the silk gland had been transplanted was raised for 3 days.
5) Collection of silk gland: The transplanted silk gland was taken out of the host. After washed with Grace medium, the silk gland was divided into middle silk gland and posterior silk gland.
6) Measurement of luciferase activity: Middle silk gland and posterior silk gland were homogenized in a passive lysis buffer (Promega) and then subjected to centrifugation. Then, luciferase activity in the supernatant was measured using a luciferase determination system (Promega).

One kind of the DNA mixed solution was introduced into 9 to 12 silk glands and then luciferase activity was measured. An average value and standard deviation (SEM) of each solution were calculated. FIG. 1 shows the results. The values were shown as relative values when the average value of the luciferase activity in middle silk gland in the silk gland into which Pser was introduced was regarded as 1.

The luciferase relative activity values in middle silk glands into which Pser, Pser+hr3, Pser+IE1, and Pser+hr3+IE1 were introduced were found to be 1, 5.2, 3.7, and 31.4, respectively. Thus, hr3 and IE1 enhance transcription activity of sericin 1 promoter even when they were used singly but it became obvious that the effect of enhancing the transcription activity became extremely high when both of hr3 and IE1 were present.

### Example 2

### Preparation of vector for producing transgenic silkworm

A double-strand oligonucleotide wherein an oligonucleotide 5'-AATTCCTTAAGCTCGAGTCGCGA-3' (SEQ ID NO: 10) phosphorylated at 5'-end and an oligonucleotide 5'-AATTTCGCGACTCGAGCTTAAGG-3' (SEQ ID NO: 11) were annealed was prepared. The double-strand oligonucleotide has restriction enzyme-recognizing sequences for AflII, XhoI, and NruI and both ends have a structure linkable to an EcoRI site. The two-strand oligonucleotide was inserted into an EcoRI site of pBac[3xP3-DsRed/pA] (Nat. Biotechnol. 21, 52-56 (2003)), which is a piggyBac vector having a red fluorescent protein (DsRed) gene expressing in eyes and nerve systems, as a marker gene to thereby insert the restriction enzyme-recognizing sequences for AflII, XhoI, and NruI into the pBac[3xP3-DsRed/pA] vector.

A DNA fragment comprising sericin 1 promoter and IE1ORF was amplified by PCR using the vector [3] of Example 1 (vector having ORF of IE1 at downstream of hr3 and sericin 1 promoter). The primers used for PCR were 5'-GAATTCAGTCGAATTTCGACTACTGCG-3' (SEQ ID NO: 12) and 5'-GAATTCCAGGAGTGGGCATACTCTTG-3' (SEQ ID NO: 13) and an EcoRI site was added to each 5'-end. Thus, EcoRI sites were added to both ends of the resulting DNA fragment. The DNA fragment was digested with EcoRI and inserted into the EcoRI site of the above piggyBac vector having AfIII, XhoI, and NruI.

Similarly, a DNA fragment comprising hr3 and sericin 1 promoter and having XhoI sites at both ends was amplified by PCR using the vector [2] of Example 1 (vector having firefly luciferase gene at downstream of hr3 and sericin 1 promoter). The primers used were 5'-CTCGAGGATATCGAATTCCTGCAGCC-3' (SEQ ID NO: 14) and 5'-CTCGAGCCCGATGATAAGACGACTATG-3' (SEQ ID NO: 15). The amplified DNA fragment was digested with XhoI and then, inserted into the XhoI site of the above piggyBac vector into which sericin 1 promoter and IE1ORF had been inserted.

Finally, using a Gateway vector conversion system (Invitrogen), a DNA fragment (Gateway cassette) containing attR1 and attR2 which are site-specific recombination sites derived from lambda phage was inserted into the NruI site of the above vector (pMSG2: FIG. 2). By inserting the DNA fragment, it becomes possible to incorporate any aimed gene to be expressed into downstream of sericin 1 promoter to which hr3 has been added utilizing the Gateway system (Invitrogen). The incorporated aimed gene is highly expressed in middle silk gland by the action of the sericin 1 promoter to which hr3 has been added. Furthermore, the expression is enhanced by the action of IE1 protein synthesized from IE1ORF incorporated into the vector. Since hr3 and sericin 1 promoter are present at upstream of IE1ORF incorporated into the vector, IE1 protein is highly expressed in middle silk gland. Therefore, the expression of the aimed gene in middle silk gland reaches a very high level.

### Example 3

### Production of transgenic silkworm secreting green fluorescent protein into sericin layer

Using a primer containing a sequence encoding a signal peptide of human calreticulin (5-CACCATGCTGCTATCCGTGCCGTTGCGGCCTCCTCGGCCTGGCC GTCGCCGTGAGCAAGGGCGAGGAG-3': SEQ ID NO: 16) and a primer containing a termination codon of EGFP (5'-TTTACTTGTACAGCTCGTCCATGC-3': SEQ ID NO: 17), a cDNA of EGFP to which a sequence encoding the signal peptide of human calreticulin was added to 5'-end was prepared by PCR using pEGFP (Clontech). The resulting cDNA fragment was incorporated into pENTR vector (Invitrogen) and inserted into pMSG2 utilizing the Gateway system to construct a vector (pSEM2) expressing secretory EGFP.

After pSEM2 was purified by cesium chloride ultracentrifugation, pSEM2 and pHA3PIG (Nat. Biotechnol. 18, 81-84 (2000)) which was a helper plasmid were mixed so that the amounts of the plasmids were 1:1. Furthermore, after ethanol precipitation, the precipitate was dissolved into an injection buffer (0.5 mM phosphate buffer pH7.0, 5 mM KCl) so that the concentration of each of pSEM and pHA3PIG was 200 µg/ml. The DNA solution was microinjected into silkworm eggs (silkworm embryos) at preblastoderm stage after 2 to 8 hours from oviposition in a liquid amount of about 15- 20 nl per egg. The microinjection was performed on 3466 eggs in total.

When the eggs into which the vector DNA had been microinjected were incubated at 25°C, 553 eggs were hatched. The hatched silkworms were continued to raise and the resulting reproductive adult worms were crossed to obtain 143 groups of F1 egg masses. By observing F1 egg masses on the 5th to 6th day from oviposition by means of a fluorescent stereoscopic microscope, eggs of transgenic silkworms emitting red fluorescence from eyes and nerve systems were screened. As a result, 7 groups of egg masses containing the eggs of transgenic silkworms can be obtained. When the resulting egg masses were hatched and raised, transgenic silkworms derived from 6 groups of the egg masses were dead before reached a silk-spinning stage but transgenic silkworms derived from 1 group of the egg mass normally made cocoons to be larvae and further emerged into adult worms having reproductive ability. Then, they were crossed with wild-type silkworms to establish a transgenic line.

The transgenic silkworm produced by pSEM2 emitted strong green fluorescence from its middle silk gland when it grew to 5th instar stage. When it reached a silk-spinning stage, it spun cocoon filaments emitting green fluorescence to make a cocoon. Thus, it was suggested that the transgenic silkworm synthesized EGFP in middle silk gland and secreted EGFP into sericin layer of the cocoon filaments. Then, the cocoon filaments were immersed in a neutral buffer containing no protein-denaturing agent, whereby extraction of EGFP was attempted. After the cocoon filaments were fragmented by scissors, the fragments were suspended in PBS containing 1% triton-X and incubated at 4°C for 48 hours. Similarly, fragments of cocoon filaments of wild-type silkworms and cocoon filaments of transgenic silkworms produced with pMOSRA-7 vector (Nat. Biotechnol. 21, 52-56 (2003)) (containing fibroin L chain, triple helix region of minicollagen, and fusion protein of EGFP in insoluble fibroin layer) were also incubated in PBS containing 1% Triton-X. The fragments of cocoon filaments were removed by centrifugation and each extract solution was observed by means of a fluorescent stereoscopic microscope. As a result, green fluorescence was detected only from the extract solution of the cocoons of the transgenic silkworms produced with pSEM2. The fact that green fluorescence was detected from the extract solution shows that EGFP secreted into the sericin layer is extracted into the neutral buffer without denaturing the steric structure.

Furthermore, Western blotting analysis was carried out by subjecting proteins contained in the extract solution to electrophoresis. The extract solution was mixed with an equal amount of SDS sample buffer and the mixture was heated at 95°C for 5 minutes. The sample was subjected to SDS polyacrylamide gel electrophoresis (Nature 227, 680-685, 1970) and the electrophoresed protein was transferred to a nitrocellulose membrane BA85 (S&S) in accordance with the method of Matsudaira et al. (J. Biol. Chem. 262, 10035-10038, 1987). After the nitrocellulose membrane was treated with a blocking solution (5% skim milk/50 mM Tris hydrochloric acid buffer pH 7.5, 150 mM NaCl) at room temperature for 1 hour, the membrane was reacted at room temperature for 1 hour with anti-EGFP polyclonal antibody (Clontech) diluted 1000 times with the blocking solution. Then, it was reacted at room temperature for 1 hour with peroxidase-labeled anti-rabbit IgG antibody (Cell Signaling Technology) diluted 3000 times with TBS (50 mM Tris hydrochloric acid buffer pH 7.5, 150 mM NaCl) containing Tween 20. Finally, proteins with which the antibody had been reacted was detected using ECL Western Blotting Detection Reagents (Amersham Biosciences). As a result, any band reactive with EGFP antibody was not detected from the extract solutions of cocoons of wild-type silkworms and cocoons of the transgenic silkworms produced with the pMOSRA-7 vector but, from the extract solution of the cocoons of the transgenic silkworms produced using pSEM2 vector, a clear band can be detected at the position of 27 kDa which is coincident with the molecular weight of EGFP (FIG. 3).

From the above results, it was confirmed that the cocoons of the transgenic silkworms produced with pSEM2 vector contained recombinant EGFP capable of being extracted with a neutral buffer containing no protein denaturing agent. Thus, it became obvious that the recombinant protein gene inserted into pMSG2 vector was not a fusion protein with silk proteins such as fibroin and could be expressed and secreted as the recombinant protein alone and further it could be extracted from the cocoons with the neutral buffer without denaturing the higher order structure of the protein.

### Example 4

### Cloning of sequence of 5' upstream region of silkworm sericin 2 gene

As for 5' upstream region of silkworm sericin 2 gene, only the sequence of base Nos. -80 to +60 when the transcription initiating point is regarded as +1 has been reported (Gene 86, 177-184, 1990) but further upper stream region of the sequence has not yet been known. Thus, 5' upstream region of sericin 2 gene whose sequence has not been determined was amplified from silkworm genome DNA by asymmetrical PCR and sequenced. The asymmetrical PCR method is a method of obtaining an unknown sequence adjacent to a known sequence by annealing toward the known sequence a primer specific to the sequence and by annealing a degenerate random primer toward the unknown sequence. In order to prevent amplification of undesired sequences, PCR was performed more than once using the known sequence-specific primer as a nested primer to refine the amplified product. A known sequence of sericin 2 gene was described in a paper of Michaille et al. (Gene 86, 177-184, 1990). With reference to the base sequence of from base No. -80 to base No. +60 when the base at the transcription initiating point was regarded as +1, three primers of GSP-A (5'-TGGGATCTTCATGATGACTCGTGTGGCTC-3': SEQ ID NO: 18), GSP-B (5'-GACAGACCACCTTTATATAGCCGGTGCCAC-3': SEQ ID NO: 19), and GSP-C (5'-GACAGTGCACGTCGGTCAAACTGTGTCAAC-3': SEQ ID NO: 20) were designed. As a template, 100 ng of a genome DNA extracted from abdomen of an adult silkworm was used. The reaction of asymmetrical PCR was carried out using APAgene Locator Kit (Bio S&T Inc.). As a degenerate random primer, enzymes, a substrate, and the like, those attached to the kit were used and all reaction conditions were determined following the protocol attached to the kit. As a result, a DNA fragment of about 1.5 kb could be amplified. The amplification product was subcloned to pCR4Blunt-TOPO vector (Invitrogen) and its base sequence was determined by the dideoxy method. The base sequence is shown as SEQ ID NO: 1.

### Industrial Applicability

As precisely described in the above, according to the invention of the present application, a polynucleotide comprising a gene expression-regulating sequence capable of expressing a recombinant protein gene in middle silk gland and having high transcription activity is provided. Furthermore, a vector containing the polynucleotide, and a transgenic silkworm produced using the vector, as well as a process for producing a recombinant protein comprising extracting the recombinant protein from cocoons produced by the transgenic silkworm are provided. Utilizing the invention, various recombinant proteins can be secreted in silkworm cocoons as not fusion proteins with silk proteins but single proteins. Furthermore, the recombinant proteins can be easily extracted from the cocoons without denaturing the proteins. Accordingly, it becomes possible to produce recombinant proteins usable in various industrial fields of medical care, foods, cosmetics, fibers, and the like in a large amount and in an easy manner.

### SEQUENCE LISTING

〈110〉 Hiroshima Industrial Promotion Organization and Biointegrence Inc.
〈120〉 Polynucleotide for-expression of recombinant protein in silk worm 〈130〉 05-F-064PCT
〈150〉 JP2004-301834 〈151〉 2004-10-15
〈160〉 20
〈170〉 Patent In version 3,1
〈210〉 1
   〈211〉 1490
   〈212〉 DNA
   〈213〉 Bombyx mori
〈400〉 1 
cagaatctac cacgatcgga aacgcgaccc actgagaaga tccggcgaga aactcagtga 60

gctgtgtcta tgggttaatt tactcgtcga gccctgttta ctgtttaggg cgacgtcgac 120

tgttaccatt cggtctacag gatcgagtgt gcattcttgt atcatcgttc tattatcacg 180

agtcattttg cgtttttcg gatcccctgg aagtcgtcgt ggcctaagag ataagaagtc 240

cggtgcattc gtgttgagcg atgcacctgt gttcgaatcc taggcgggta ccaatttttc 300

taatgaatta cgtacccaac aaattgttca cgattgcctt ccacggtgaa ggaataacat 360

cgtgcaataa aagtgaaacc cgcaaaatcc ggtgctttta agcttttcaa gcaccggtca 420

ccatcctcgt tgaactcatc gatctacaag cgatctaatc tatagaccca atccactaag 480

atctcaccgg atcttctcag tggttcgcat tccagtggta gattcaattc gctgctcttg 540

ctagggctag tgttagcaaa ttccttcggg ttaagcccga gagctcacct atccgtccgc 600

gctaagctgg aaaagcccct taagctgttt tttttgta tagcctttat tgctaatact 660

aaacaataac taataatttt acatacagta acaaattgtt ttaacttaaa tctaatacat 720
 
cggatttccc ggttcagtga tcagcgtgtc ctgtgacaca taggcctctt ccagctgctt 780

tcatttttct ctattggtag cttttcttga ccagattgtc tctccaatca tcttgatatc 840

gtctgtccat cttctagctt gcctggctct tttcctttaa accaggggtc gtgaattcaa 900

tcctcacagg aagccgggat taggtgggag aatatagttc cgatgttttg aatgctttat 960

attttctgtg gtcgaaaatg atactagagc tacgcgtcga caattgaata ttatgctaac 1020

taccctctat ttattaaaag acttttacga ttcatttcgc acagaaccaa tcgactgggt 1080

ttagaggttt agcagtttgt tgaatgaact cgttttcatc ttcacgatta gaggatccca 1140

ggtgttaggt aaaggatatt ctagattgca ggagattttt cataaataat cacgcgatgg 1200

agcggtaatc agccaacata gtcgatcggc atcattattg gagaccaaac aacacttcag 1260

ttatccaagc gcgtcttaag tcgcattcgg ataatcttga atagcctgga agtgaatttt 1320

taaaaagttt gtctcgaaca aacatcaatt actttgtaat tgaaccgaaa aaagaggata 1380

aacattatta gcattcgttg taatgaaata taatgttgac acagtttgac cgacgtgcac 1440

tgtcttttgt ggcaccggct atataaaggt ggtctgtccg ttctgagcca 1490
〈210〉 2
   〈211〉 27
   <212> DNA
   〈213〉 Artificial
〈220〉
   <223> Description of artificial sequence: synthetic oligonucleotide
<400> 2
   gctagcagtc gaatttcgac tactgcg 27
〈210〉 3
   〈211〉 27
   〈212〉 DNA
   〈213〉 Artificial
〈220〉
   <223> Description of artificial sequence: synthetic oligonucleotide
<400> 3
   gctagccccg atgataagac gactatg 27
〈210〉 4
   〈211〉 22
   〈212〉 DNA
   <213> Artificial
<220>
   <223> Description of artificial sequence: synthetic oligonucleotide
〈400〉 4
   cggaatctat gttacggact tc 22
<210> 5
   〈211〉 26
   〈212〉 DNA
   〈213〉 Artificial
〈220〉
   〈223〉 Description of artificial sequence: synthetic oligonucleotide
<400> 5
   gagctcgata tcgaattcct gcagcc 26
〈210〉 6
   〈211〉 26
   〈212〉 DNA
   〈213〉 Artificial
〈220〉
   〈223〉 Description of artificial sequence: synthetic oligonucleotide
<400> 6
   ggatcccaac caaacgacta tgacgc 26
〈210〉 7
   〈211〉 20
   <212> DNA
   〈213〉 Artificial
〈220〉
   〈223〉 Description of artificial sequence: synthetic oligonucleotide
〈400〉 7
   caggagtggg catactcttg 20
〈210〉 8
   〈211〉 33
   〈212〉 DNA
   <213> Artificial
〈220〉
   〈223〉 Description of artificial sequence: synthetic oligonucleotide
〈400〉 8
   ggatccgagc tcagtcgaat ttcgactact gcg 33
〈210〉 9
   〈211〉 33
   〈212〉 DNA
   〈213〉 Artificial
〈220〉
   <223> Description of artificial sequence: synthetic oligonucleotide
〈400〉 9
   ggatccgcta gccccgatga taagacgact atg 33
〈210〉 10
   〈211〉 23
   〈212〉 DNA
   〈213〉 Artificial
〈220〉
   <223> Description of artificial sequence: synthetic oligonucleotide
〈400〉 10
   aattccttaa gctcgagtcg cga 23
〈210〉 11
   〈211〉 23
   〈212〉 DNA
   <213> Artificial
<220>
   <223> Description of artificial sequence: synthetic oligonucleotide
〈400〉 11
   aatttcgcga ctcgagctta agg 23
〈210〉 12
   <211> 27
   〈212〉 DNA
   〈213〉 Artificial
〈220〉
   〈223〉 Description of artificial sequence: synthetic oligonucleotide
〈400〉 12
   gaattcagtc gaatttcgac tactgcg 27
<210> 13
   〈211〉 26
   〈212〉 DNA
   〈213〉 Artificial
〈220〉
   〈223〉 Description of artificial sequence: synthetic oligonucleotide
〈400〉 13
   gaattccagg agtgggcata ctcttg 26
〈210〉 14
   〈211〉 26
   〈212〉 DNA
   〈213〉 Artificial
<220>
   <223> Description of artificial sequence: synthetic oligonucleotide
〈400〉 14
   ctcgaggata tcgaattcct gcagcc 26
〈210〉 15
   〈211〉 27
   〈212〉 DNA
   <213> Artificial
〈220〉
   〈223〉 Description of artificial sequence: synthetic oligonucleotide
〈400〉 15
   ctcgagcccg atgataagac gactatg 27
〈210〉 16
   〈211〉 73
   〈212〉 DNA
   〈213〉 Artificial
〈220〉
   <223> Description of artificial sequence: synthetic oligonucleotide
〈400〉 16 
caccatggtg ctatccgtgc cgttgctgct cggcctcctc ggcctggccg tcgccgtgag 60

caagggcgag gag 73
〈210〉 17
   〈211〉 24
   〈212〉 DNA
   〈213〉 Artificial,
〈220〉
   〈223〉 Description of artificial sequence: synthetic oligonucleotide
〈400〉 17
   tttacttgta cagctcgtcc atgc 24
<210> 18
   <211> 29
   〈212〉 DNA
   〈213〉 Artificial
〈220〉
   〈223〉 Description of artificial sequence: synthetic oligonucleotide
〈400〉 18
   tgggatcttc atgatgactc gtgtggctc 29
〈210〉 19
   〈211〉 30
   〈212〉 DNA
   〈213〉 Artificial
〈220〉
   〈223〉 Description of artificial sequence: synthetic oligonucleotide
〈400〉 19
   gacagaccac ctttatatag ccggtgccac 30
〈210〉 20
   〈211〉 30
   〈212〉 DNA
   〈213〉 Artificial
〈220〉
   〈223〉 Description of artificial sequence: synthetic oligonucleotide
〈400〉 20
   gacagtgcac gtcggtcaaa ctgtgtcaac 30

## Claims

1. A polynucleotide comprising a polynucleotide corresponding to a promoter region of sericin 1 gene or sericin 2 gene functionally linked with a polynucleotide corresponding to baculovirus homologous regions capable of being functionally linked to a structural gene for a recombinant protein in an expression cassette for expressing the recombinant protein in silkworm middle silk gland.

2. The polynucleotide of claim 1, wherein the polynucleotide corresponding to a promoter region of sericin 2 gene has a nucleotide sequence of SEQ ID NO: 1 or a part thereof.

3. An expression cassette for expressing a recombinant protein in silkworm middle silk gland, wherein the polynucleotide of claim 1 or 2 and a structural gene for the recombinant protein are functionally linked.

4. A polynucleotide promoting the transcription activity possessed by the polynucleotide of claim 1 or 2, in which a polynucleotide corresponding to a promoter region of sericin 1 gene or sericin 2 gene is functionally linked with a polynucleotide encoding baculovirus IE1 protein.

5. The polynucleotide of claim 4, wherein the polynucleotide corresponding to a promoter region of sericin 2 gene has the nucleotide sequence of SEQ ID NO: 1 or a part thereof.

6. A polynucleotide, in which a polynucleotide corresponding to baculovirus homologous regions is further linked with the polynucleotide of claim 4 or 5 corresponding to a promoter region of sericin 1 gene or sericin 2 gene functionally linked with a polynucleotide encoding baculovirus IE1 protein.

7. An expression vector having the expression cassette of claim 3.

8. The expression vector of claim 7, wherein the expression cassette is sandwiched between one pair of inverted repeat sequences of an insect-derived DNA-type transposon.

9. A vector having the polynucleotide of claim 4 or 5 or the polynucleotide of claim 6.

10. The vector of claim 9, wherein the polynucleotide is sandwiched between one pair of inverted repeat sequences of an insect-derived DNA-type transposon.

11. A vector having the expression cassette of claim 3 and the polynucleotide of claim 4, 5, or 6.

12. The vector of claim 11, wherein the expression cassette and the polynucleotide are sandwiched between one pair of inverted repeat sequences of an insect-derived DNA-type transposon.

13. A transgenic silkworm, which possesses the expression cassette of claim 3 in its genome and expresses a recombinant protein in its middle silk gland.

14. A transgenic silkworm, which has the polynucleotide of claim 4, 5, or 6 in its genome and expresses baculovirus IE1 protein in its middle silk gland.

15. A transgenic silkworm, which has the expression cassette of claim 3 and the polynucleotide of claim 4, 5, or 6 in its genome and expresses a recombinant protein in its middle silk gland.

16. A process for producing a recombinant protein comprising extracting the recombinant protein from cocoons of the transgenic silkworm of claim 13.

17. A process for producing a recombinant protein comprising extracting the recombinant protein from cocoons of the transgenic silkworm of claim 15.

18. A polynucleotide corresponding to a promoter region of sericin 2 gene, which comprises the nucleotide sequence of SEQ ID NO: 1 or a part thereof.

## Patentansprüche

1. Polynukleotid mit einem Polynukleotid, das einer Promotorregion von Sericin-1-Gen oder Sericin-2-Gen entspricht, das mit einem Polynukleotid funktional verbunden ist, das homologen Baculovirus-Regionen entspricht, die mit einem Strukturgen für ein rekombinantes Protein in einer Expressionskassette zum Exprimieren des rekombinanten Proteins in der Seidenraupen-Mittelspinndrüse funktional verbunden werden können.

2. Polynukleotid nach Anspruch 1, wobei das Polynukleotid, das einer Promotorregion von Sericin-2-Gen entspricht, eine Nukleotidsequenz von SEQ ID NR.: 1 oder einen Teil davon aufweist.

3. Expressionskassette zum Exprimieren eines rekombinanten Proteins in der Seidenraupen-Mittelspinndrüse, wobei das Polynukleotid nach Anspruch 1 oder 2 und ein Strukturgen für das rekombinante Protein funktional verbunden sind.

4. Polynukleotid, das die Transkriptionsaktivität fördert, die das Polynukleotid nach Anspruch 1 oder 2 besitzt, wobei ein Polynukleotid, das einer Promotorregion von Sericin-1-Gen oder Sericin-2-Gen entspricht, mit einem Polynukleotid, das Baculovirus-IE1-Protein codiert, funktional verbunden ist.

5. Polynukleotid nach Anspruch 4, wobei das Polynukleotid, das einer Promotorregion von Sericin-2-Gen entspricht, die Nukleotidsequenz von SEQ ID NR.: 1 oder einen Teil davon aufweist.

6. Polynukleotid, in dem ein Polynukleotid, das homologen Baculovirusregionen entspricht, außerdem mit dem Polynukleotid nach Anspruch 4 oder 5 verbunden ist, das einer Promotorregion von Sericin-1-Gen oder Sericin-2-Gen entspricht, das mit einem Polynukleotid, das Baculovirus-IE1-Protein codiert, funktional verbunden ist.

7. Expressionsvektor mit der Expressionskassette nach Anspruch 3.

8. Expressionsvektor nach Anspruch 7, wobei die Expressionskassette zwischen ein Paar von invertierten Wiederholungssequenzen eines von Insekten abgeleiteten Transposons vom DNA-Typ eingelegt ist.

9. Vektor mit dem Polynukleotid nach Anspruch 4 oder 5 oder dem Polynukleotid nach Anspruch 6.

10. Vektor nach Anspruch 9, wobei das Polynukleotid zwischen ein Paar von invertierten Wiederholungssequenzen eines von Insekten abgeleiteten Transposons vom DNA-Typ eingelegt ist.

11. Vektor mit der Expressionskassette nach Anspruch 3 und dem Polynukleotid nach Anspruch 4, 5 oder 6.

12. Vektor nach Anspruch 11, wobei die Expressionskassette und das Polynukleotid zwischen ein Paar von invertierten Wiederholungssequenzen eines von Insekten abgeleiteten Transposons vom DNA-Typ eingelegt sind.

13. Transgene Seidenraupe, die die Expressionskassette nach Anspruch 3 in ihrem Genom besitzt und ein rekombinantes Protein in ihrer Mittelspinndrüse exprimiert.

14. Transgene Seidenraupe, die das Polynukleotid nach Anspruch 4, 5 oder 6 in ihrem Genom aufweist und Baculovirus-IE1-Protein in ihrer Mittelspinndrüse exprimiert.

15. Transgene Seidenraupe, die die Expressionskassette nach Anspruch 3 und das Polynukleotid nach Anspruch 4, 5 oder 6 in ihrem Genom aufweist und ein rekombinantes Protein in ihrer Mittelspinndrüse exprimiert.

16. Verfahren zur Herstellung eines rekombinanten Proteins, das das Extrahieren des rekombinanten Proteins aus Kokons der transgenen Seidenraupe nach Anspruch 13 umfasst.

17. Verfahren zur Herstellung eines rekombinanten Proteins, das das Extrahieren des rekombinanten Proteins aus Kokons der transgenen Seidenraupe nach Anspruch 15 umfasst.

18. Polynukleotid, das einer Promotorregion von Sericin-2-Gen entspricht, das die Nukleotidsequenz von SEQ ID NR.: 1 oder einen Teil davon aufweist.

## Revendications

1. Polynucléotide comportant un polynucléotide correspondant à une région promotrice de gène de séricine 1 ou de gène de séricine 2 liée de manière fonctionnelle à un polynucléotide correspondant à des régions homologues baculovirus pouvant être liées de manière fonctionnelle à un gène structurel pour une protéine recombinante dans une cassette d'expression afin d'exprimer la protéine recombinante dans la glande séricigène d'un ver à soie.

2. Polynucléotide selon la revendication 1, dans lequel le polynucléotide correspondant à une région promotrice de gène de séricine 2 a une séquence de nucléotides de NO ID SEQ:1 ou une partie de celle-ci.

3. Cassette d'expression pour exprimer une protéine recombinante dans une glande de soie séricigène d'un ver à soie, dans laquelle le polynucléotide de la revendication 1 ou 2 et un gène structurel pour la protéine recombinante sont liés de manière fonctionnelle.

4. Polynucléotide promouvant l'activité de transcription possédée par le polynucléotide de la revendication 1 ou 2, dans lequel un polynucléotide correspondant à une région promotrice de gène de séricine 1 ou de gène de séricine 2 est lié de manière fonctionnelle à un polynucléotide codant une protéine IE1 baculovirus.

5. Polynucléotide selon la revendication 4, dans lequel le polynucléotide correspondant à une région promotrice de gène de séricine 2 a la séquence de nucléotides de NO ID SEQ:1 ou une partie de celle-ci.

6. Polynucléotide, dans lequel un polynucléotide correspondant à des régions homologues baculovirus est en outre lié au polynucléotide de la revendication 4 ou 5 correspondant à une région promotrice de gène de séricine 1 ou de gène de séricine 2 liée de manière fonctionnelle à un polynucléotide codant une protéine IE1 baculovirus.

7. Vecteur d'expression ayant la cassette d'expression de la revendication 3.

8. Vecteur d'expression selon la revendication 7, dans lequel la cassette d'expression est enserrée entre une paire de séquences répétées inversées d'un transposon de type ADN dérivé d'insecte.

9. Vecteur ayant le polynucléotide de la revendication 4 ou 5 ou le polynucléotide de la revendication 6.

10. Vecteur selon la revendication 9, dans lequel le polynucléotide est enserré entre une paire de séquences répétées inversées d'un transposon de type ADN dérivé d'insecte.

11. Vecteur ayant la cassette d'expression de la revendication 3 et le polynucléotide de la revendication 4, 5 ou 6.

12. Vecteur selon la revendication 11, dans lequel la cassette d'expression et le polynucléotide sont enserrés entre une paire de séquences répétées inversées d'un transposon de type ADN dérivé d'insecte.

13. Ver à soie transgénique, lequel possède la cassette d'expression de la revendication 3 dans son génome et exprime une protéine recombinante dans sa glande séricigène.

14. Ver à soie transgénique, lequel a le polynucléotide de la revendication 4, 5 ou 6 dans son génome et exprime une protéine IE1 baculovirus dans sa glande séricigène.

15. Ver à soie transgénique, lequel a la cassette d'expression de la revendication 3 et le polynucléotide de la revendication 4, 5, ou 6 dans son génome et exprime une protéine recombinante dans sa glande séricigène.

16. Procédé pour produire une protéine recombinante comportant l'extraction de la protéine recombinante de cocons du ver à soie transgénique de la revendication 13.

17. Procédé pour produire une protéine recombinante comportant l'extraction de la protéine recombinante de cocons du ver à soie transgénique de la revendication 15.

18. Polynucléotide correspondant à une région promotrice de gène de séricine 2, laquelle comporte la séquence de nucléotides de NO ID SEQ:1 ou une partie de celle-ci.
